# EUROPÄISCHE PATENTSCHRIFT

(11) **EP 0 579 076 B1**
(45) Veröffentlichungstag und Bekanntmachung des Hinweises auf die Patenterteilung: **10.09.1997**
(21) Anmeldenummer: 93110693.4
(22) Anmeldetag: 05.07.1993
(51) Int. Cl.: A61F 9/06

(54) **Elektrooptische Blendschutzvorrichtung für Schweisser**
Electro optical glare protection for welding
Ecran électro-optique de protection pour soudeur

(30) Priorität: 13.07.1992 CH 2196/92
(43) Veröffentlichungstag der Anmeldung: 19.01.1994
(73) Patentinhaber: XELUX HOLDING AG, 6300 Zug (CH)
(72) Erfinder: Gunz, Stefan, CH-8820 Wädenswil (CH); Werthmüller, René, CH-2563 Ipsach (CH); Ghisleni, Livio, CH-8832 Wilen b. Wollerau (CH)
(74) Vertreter: Ritscher, Thomas, Dr.

(56) Entgegenhaltungen:
- EP-A- 0 027 518
- EP-A- 0 335 056
- EP-A- 0 349 665
- DE-A- 3 536 678
- US-A-RE29684 (MACK)

## Beschreibung

Die vorliegende Erfindung betrifft eine Blendschutzvorrichtung gemäss Präambel des vorliegenden Anspruchs 1.

Blendschutzvorrichtungen dieser Art sind dem Fachmann hinlänglich bekannt. Diese umfassen im wesentlichen eine Filteranordnung mit mindestens einer Flüssigkristallzelle, mindestens einen Polarisator und einen Analysator, sowie verschiedene passive Lichtfilter, insbesondere IR- und UV-Sperrfilter. Dabei wird die Flüssigkristallzelle von einem als Speisestromquelle arbeitenden optoelektrischen Wandler getrieben, wobei das Betriebssignal für die Flüssigkristallzelle entweder manuell oder von einem optischen Sensor, insbesondere diesem optoelektrischen Wandler, geregelt wird. Ziel dieser Filteranordnungen ist es, eine möglichst gleichbleibende Beleuchtungsintensität für das Auge zu erreichen, während die Objektbeleuchtung starken Schwankungen ausgesetzt ist.

So wird bspw. in der EP-A-335 056 ein Lichtfilter beschrieben mit selbsttätiger Regelung der optischen Transmission, wobei ein erster Sensor vor der Flüssigkristallzelle und ein zweiter Sensor hinter der Flüssigkristallzelle, resp. hinter einem UV-Sperrfilter, angeordnet ist. Bei dieser Filteranordnung werden die Signale dieser beiden Sensoren derart geschaltet, dass IR-Strahlungsquellen das Regelsignal für die Flüssigkristallzelle nicht beeinflussen.
Die US-RE-29 684 betrifft eine Lichtschutzanordnung für Schweisser, dessen Flüssigkristallzelle mit Hilfe einer elektronischen Schaltung aktiviert wird. Diese elektronische Schaltung reagiert auf Signale, die bei der Erzeugung des Schweissbogens auftreten, insbesondere auf den Startfunken, auf die IR-Strahlung der Schweisselektrode, auf den Startstrom der Schweissmaschine, auf elektromagnetische oder hochfrequente Felder des Elektrokabels der Schweissmaschine.

Diese und ähnliche Filteranordnung weisen für den praktischen Einsatz erhebliche Nachteile auf. Insbesondere ist es in der Praxis dringend erwünscht, die Qualität der Schweissflamme, d.h. deren unterschiedliche Helligkeit und Färbung zu erkennen. Eine Vorrichtung welche darauf abzielt, Helligkeitsänderungen generell auszugleichen, lässt keine diesbezüglichen Qualitätsmerkmale der Schweissflamme erkennen, insbesondere erkennt der geübte Schweisser an der Helligkeit der Schweissflamme die für die Bearbeitung geeignete Temperatur und/oder die Konstanz der Flammbogenlänge.

Weitere wesentliche Nachteile der bekannten Vorrichtungen bestehen darin, dass Störlicht, z.B. das Flackern von Lichtröhren oder beliebige Helligkeitsunterschiede im Umgebungslicht ebenfalls erfasst und durch entsprechendes Abdunkeln der Sichtscheibe kompensiert werden. Damit wird aber wiederum die Beurteilung der Flammenqualität erheblich beeinträchtigt, wenn nicht sogar verunmöglicht.

Weitere Störquellen sind in allen Vorrichtungen zu sehen, welche nicht-optisch detektierbare, bspw. elektrisch oder magnetisch detektierbare Störfelder oder elektromagnetisch detektierbare Wechselfelder, insbesondere im Wellenlängenbereich des detektierten sichtbaren Lichtes erzeugen, wie sie beispielsweise auch von Halogen- oder Leuchtstoffröhren emittiert werden. Insbesondere erweisen sich die für das Schweissen verwendeten hohen strom- und spannungserzeugenden Schweissmaschinen als wesentliche Störquellen.

Ein weiteres Problem bei der Verwendung der bekannten Filteranordnungen besteht darin, dass diese nur dann verdunkeln, wenn eine Helligkeitsänderung detektiert wird, wie sie beim Hochfrequenzschweissen auftritt.

Es ist aber garade das Ziel der modernen Schweissgerätehersteller mit ihren Geräten möglichst ruhige und immer kleinere Schweissflammen erzeugen zu können. Damit sind die herkömmlichen Filteranordnungen nicht mehr geeignet, den gesetzlich vorgeschriebenen Blendschutzverordnungen generell zu genügen.

Es ist darum Aufgabe der vorliegenden Erfindung eine für alle Anwendungsbereiche einsetzbare und unter allen Betriebsbedingungen , d.h. für jede Schweissart, einwandfrei und zuverlässig arbeitende Blendschutzvorrichtung zu schaffen, welche die Nachteile der bekannten Vorrichtung nicht aufweist. Insbesondere soll eine universelle Blendschutzvorrichtung geschaffen werden, welche nicht auf elektromagnetische Störfelder reagiert und auch bei ruhigem und wenig intensivem Schweissflammlicht verwendbar ist.

Diese Aufgabe wird erfindungsgemäss durch eine Blendschutzvorrichtung mit den Merkmalen des Anspruchs 1 gelöst, welche insbesondere optische und nicht-optische Sensoren in geeigneten Kombinationen logisch miteinander verknüpft.

Insbesondere ist neben dem optischen Sensor ein als Antenne wirkender Detektor für nicht-optische Signale vorgesehen. Die Signale des optischen Sensors und des nicht-optischen Detektors werden in geeigneter Weise kombiniert, wobei die entsprechenden Signale logisch miteinander verknüpft werden, um die Flüssigkristallzelle zu schalten. Durch geeignete Vorwahl - je nach Verwendungszweck - und entsprechende Programmierung einer Logikschaltung, z.B. eines Mikroprozessors lassen sich eine Vielzahl von logischen Verknüpfungen schaffen.

Insbesondere können die Detektorsignale drahtgebunden und/oder drahtlos ebensogut übermittelt und bearbeitet werden, wie mit Faseroptik.

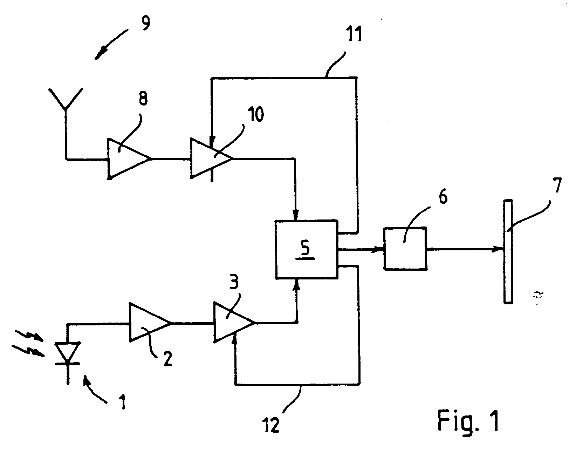

Im folgenden soll anhand der Figur ein Ausführungsbeispiel näher erläutert werden.

Die vorliegende Figur zeigt ein Blockschema für eine elektronische Schaltung wie sie beispielsweise für eine erfindungsgemässe Blendschutzvorrichtung geeignet ist. Dabei wird das von einem Fotodetektor (1) erzeugte Signal von einer Verstärkerschaltung (2) verstärkt und digitalisiert und einem Schwellwertschalter (3) zugeführt. Das von diesem Schwellwertschalter (3) erzeugte Signal wird einem erfindungsgemässen Schalter (5) zugeführt, welcher im wesentlichen einen LCD-Treiber (6) aktiviert, an welchem eine Flüssigkristallzelle (7) angeschlossen ist. Erfindungsgemäss weist diese Blendschutzvorrichtung mindestens eine weitere Detektorschaltung (9) auf, insbesondere eine Antenne für elektromagnetische Wechselfelder, deren Signal über eine Verstärkerschaltung (8) und einen Schwellwertschalter (10) ebenfalls dem genannten Schalter (5) zugeführt wird. Dieser Schalter (5) kann die detektierten Signale in gewünschter Weise, insbesondere in manuell wählbarer Weise, verarbeiten, kann elektrisch gesteuert sein oder mit vorprogrammierten Mikroprozessoren arbeiten. Insbesondere soll dieser Schalter (5) die eingehenden Detektor- resp. Sensorsignale auch in zeitlich geregelter Abfolge bearbeiten können, beispielsweise um die Sensibilität der Blendschutzvorrichtung zu variieren, wie dies beispielsweise nach dem intensiven Startfunken beim Schweissen erwünscht ist oder um zwischen optischer und nicht-optischer Detektion wählen zu können. Es versteht sich für den Fachmann die Signale des Schalters (5) auch über separate Leitungen (11,12) zurückzukoppeln.

Es ist durchaus denkbar andere Dektektoren, wie sie heute bereits vielfältig in der Sensorentechnik bekannt sind zu verwenden. Insbesondere können in der vorliegenden Vorrichtung auch Wärmedetektoren eingesetzt werden, welche Temperaturfluktuationen, Ultraschall- oder Mikrowellenfelder auflösen können.

Diese Universalität ermöglicht somit die jeweiligen Detektoren unabhängig voneinander zu postitionieren, resp. so auszubilden, dass diese vom Anwender selbst positionierbar sind.

## Patentansprüche

1. Elektrooptische Blendschutzvorrichtung für Schutzbrillen, Schutzhelme oder Schutzmasken mit mindestens einem optischen Detektor (1), insbesondere einer Fotodiode, mit einer mindestens eine Flüssigkristallzelle (7) umfassenden elektrooptischen Blendschutzscheibe und mit einer elektronischen Schaltung (6) zur Einstellung der optischen Transmission dieser Blendschutzscheibe und mindestens einem nicht-optischen Detektor (9), dadurch gekennzeichnet, dass der elektronischen Schaltung (6) zur Einstellung der optischen Transmission der Blendschutzscheibe ein Schalter (5) vorgeschaltet ist, welcher die von diesen Detektoren erzeugten Signale in gewünschter Weise logisch miteinander verknüpft und der elektronischen Schaltung ein entsprechendes Schaltsignal abgibt.

2. Blendschutzvorrichtung nach Anspruch 1, dadurch gekennzeichnet, dass der Schalter (5) die von den Detektoren (1, 9) erzeugten Signale zeitlich miteinander verknüpft.

3. Blendschutzvorrichtung nach einem der Ansprüche 1 oder 2, dadurch gekennzeichnet, dass der nicht-optische Detektor (9) eine als elektromagnetischer Detektor, insbesondere als Antenne ausgebildete elektronische Schaltung umfasst.

4. Blendschutzvorrichtung nach einem der Ansprüche 1 oder 2, dadurch gekennzeichnet, dass der nicht-optische Detektor (9) für eine drahtgebundene, insbesondere eine faseroptische und/oder drahtlose Erfassung der Signale eines Schweissgerätes geeignet ist.

5. Blendschutzvorrichtung nach Anspruch 4, dadurch gekennzeichnet, dass der Detektor (9) für die Detektion des Schweissstroms, resp. der Schweissspannung des Schweissgerätes geeignet ist.

6. Blendschutzvorrichtung nach einem der Ansprüche 1 oder 2, dadurch gekennzeichnet, dass der Detektor (9) ein Temperaturfluktuationssensor ist.

7. Blendschutzvorrichtung nach einem der Ansprüche 1 oder 2, dadurch gekennzeichnet, dass für die Einstellung der Sensitivität der Blendschutzvorrichtung und für die Wahl zwischen optischer und nicht-optischer Detektion der Schalter (5) manuell einstellbar ist.

8. Blendschutzvorrichtung nach einem der Ansprüche 1 oder 2, dadurch gekennzeichnet, dass für die Einstellung der Sensitivität der Blendschutzvorrichtung und für die Wahl zwischen optischer und nicht-optischer Detektion der Schalter (5) elektrisch gesteuert ist.

9. Blendschutzvorrichtung nach einem der Ansprüche 1 oder 2, dadurch gekennzeichnet, dass für die Einstellung der Sensitivität der Blendschutzvorrichtung und für die Wahl zwischen optischer und nicht-optischer Detektion der Schalter (5) mindestens einen vorprogrammierten Mikroprozessor aufweist.

10. Blendschutzvorrichtung nach einem der Ansprüche 1 oder 2, dadurch gekennzeichnet, dass mindestens einer der Detektoren (1, 9) lageunabhängig und/oder frei positionierbar ist.

## Claims

1. Electro-optical glare protection device for use with protective glasses, protective helmets or protective masks comprising at least one optical detector (1), in particular a photodiode, further comprising an electro-optical glare protection plate incorporating at least one liquid crystal cell (7) and an electronic circuit (6) for adjusting the optical transmission of this glare protection plate, and at least one non-optical detector (9), characterized in that a switch (5) is connected before the electronic circuit (6) for adjusting the optical transmission of the glare protection plate, said switch (5) logically linking the signals generated by these detectors with each other in an intended manner, and sending a corresponding switch signal to the electronic circuit (6).

2. Glare protection device according to claim 1, characterized in that the switch (5) links the signals produced by the detectors in time dependent manner with each other.

3. Glare protection device according to one of claims 1 or 2, characterized in that the non-optical detector (9) incorporates an electronic circuit configured as electromagenetic detector, and especially configured as an antenna.

4. Glare protection device according to one of claims 1 or 2, characterized in that the non-optical detector (9) is suitable for a wired detection, in particular by optical fibers, of the signals from a welding apparatus, and/or a wireless detection of these signals.

5. Glare protection device according to claim 4, characterized in that the detector (9) is suitable for the detection of the welding current or the welding voltage of the welding apparatus.

6. Glare protection device according to one of claims 1 or 2, chacterized in that the detector (9) is a temperature fluctuation sensor.

7. Glare protection device according to one of claims 1 or 2, characterized in that for the adjustment of the sensitivity of the glare protection device and for the selection between optical and non-optical detection, the switch (5) is manually controllable.

8. Glare protection device according to one of claims 1 or 2, characterized in that for the adjustment of the sensitivity of the glare protection device and for the selection between optical and non-optical detection, the switch (5) is controlled electrically.

9. Glare protection device according to one of claims 1 or 2, characterized in that for the adjustment of the sensitivity of the glare protection device and for the selection between optical and non-optical detection, the switch (5) incorporates at least one preprogrammed microprocessor.

10. Glare protection device according to one of claims 1 or 2, characterized in that at least one of the detectors (1, 9) is independent of position and/or can be positioned at will.

## Revendications

1. Écran de protection contre l'éblouissement pour lunette de protection, casque de protection ou masque de protection comprenant au moins un détecteur optique (1), en particulier une diode photo-électrique, une vitre de protection de protection contre l'éblouissement comprenant au moins un cellule à cristaux liquides (7), et un circuit électronique de réglage de la transmission optique de cette vitre de protection et au moins un détecteur non-optique (9), caractérisé en ce qu'un circuit (5) est disposé en amont du circuit électronique (6) de réglage, que ce circuit (5) combine logiquement de manière souhaitée les signaux émis par les détecteurs entre-eux et fournit au circuit électronique un signal correspondant.

2. Écran de protection contre l'éblouissement selon la revendication 1, caractérisé en ce que le circuit (5) combine chronologiquement les signaux émis par les détecteurs (1, 9).

3. Écran de protection contre l'éblouissement selon l'une des revendications 1 ou 2, caractérisé en ce que le détecteur non-optique (9) comprend un circuit électronique conformé en tant que détecteur électromagnétique, en particulier en tant qu'antenne.

4. Écran de protection contre l'éblouissement selon l'une des revendications 1 ou 2, caractérisé en ce que le détecteur non-optique (9) est adapté à la détection des signaux d'un appareil de soudage par l'intermédiaire de câbles de connections, en particulier de fibres optiques, ou par radio.

5. Écran de protection contre l'éblouissement selon la revendication 4, caractérisé en ce que le détecteur (9) est adapté à la détection de l'intensité, respectivement de la tension de soudage de l'appareil de soudage.

6. Écran de protection contre l'éblouissement selon l'une des revendications 1 ou 2, caractérisé en ce que le détecteur (9) est un capteur de fluctuation de la température.

7. Écran de protection contre l'éblouissement selon l'une des revendications 1 ou 2, caractérisé en ce que le circuit (5) est réglable manuellement pour le réglage de la sensibilité de l'écran de protection contre l'éblouissement et pour le choix entre détection optique et non-optique.

8. Écran de protection contre l'éblouissement selon l'une des revendications 1 ou 2, caractérisé en ce que le circuit (5) est commandé électriquement pour le réglage de la sensibilité de l'écran de protection contre l'éblouissement et pour le choix entre détection optique et non-optique.

9. Écran de protection contre l'éblouissement selon l'une des revendications 1 ou 2, caractérisé en ce que le circuit (5) comprend au moins un microprocesseur préprogrammé pour le réglage de la sensibilité de l'écran de protection contre l'éblouissement et pour le choix entre détection optique et non-optique.

10. Écran de protection contre l'éblouissement selon l'une des revendications 1 ou 2, caractérisé en ce qu'au moins un des détecteurs (1, 9) est insensible à son positionnement ou susceptible d'être positionné librement.
